(19) Europäisches Patentamt
European Patent Office
Office européen des brevets

(11) Publication number : 0 345 951 B1

(12) EUROPEAN PATENT SPECIFICATION

(45) Date of publication of patent specification : 10.03.93 Bulletin 93/10

(51) Int. Cl.$^5$ : A61K 31/557

(21) Application number : 89304723.3

(22) Date of filing : 10.05.89

# (54) Tracheobronchodilator.

(30) Priority : 11.05.88 JP 115409/88

(43) Date of publication of application : 13.12.89 Bulletin 89/50

(45) Publication of the grant of the patent : 10.03.93 Bulletin 93/10

(84) Designated Contracting States : AT BE CH DE ES FR GB GR IT LI LU NL SE

(56) References cited :
US-A- 4 022 821
US-A- 4 038 308
US-B- 491 711
CHEMICAL ABSTRACTS, vol. 74, 1971, page 78, abstract 120819y Columbus, Ohio, US J.NAKANO
J.PHARM. PHARMAC., vol. 29, May 10, 1977, pages 752-755 P. YONG LO
BR. J. PHARMAC., vol. 54, 1975, pages 397-399, D.J. CRUTCHLEY et al.

(73) Proprietor : Kabushiki Kaisha Ueno Seiyaku Oyo Kenkyujo
4-8, 2-chome, Koraibashi Chuo-ku
Osaka-shi Osaka-fu (JP)

(72) Inventor : Ryuzo, Ueno
10-27, Nango-cho Nishinomiya-shi
Hyogo-Ken (JP)
Inventor : Ryuji, Ueno
7-29, Misaku-cho Nishinomiya-shi
Hyogo-Ken (JP)
Inventor : Tomio, Oda
B-203, 1-29 Suzukakedai
Sandi-shi Hyogo-Ken (JP)

(74) Representative : Atkinson, Peter Birch et al
MARKS & CLERK Suite 301 Sunlight House
Quay Street
Manchester M3 3JY (GB)

EP 0 345 951 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a tracheobronchodilator containing a 15-keto-prostaglandin E derivative. In the present specification a series of prostaglandins and their derivatives are abbreviated to PGs. Accordingly, a series of compounds belonging to 15-keto -prostaglandin E such as 15-keto-prostaglandin $E_1$, $E_2$, esters, salts, or others having substituents is generically called 15-keto-PGEs. Term "$PGE_1$s" means a series of compounds belonging to $PGE_1$.

Prostaglandin is a generic name for carboxylic acids having a variety of physiological activities, which are found in tissues or internal organs of humans or animals. PGs have a basic skeleton of a prostanoic acid represented by following formula:

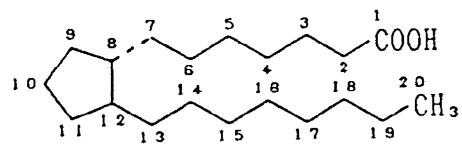

There have been provided various kinds of synthetic PGs modified on one or more carbon atom(s) of the skeleton.

PGs are classified into PGAs, PGBs, PGCs, PGDs, PGEs, PGFs, PGIs, and PGJs and so on according to the structure or the five membered ring in the above skeleton. Further, they are classified into $PG_1$s in which the bond between 5- and 6-positions is a single bond; $PG_2$s in which the bond between 5- and 6-positions is a double bond; and $PG_3$s in which the bonds between 5- and 6-positions, and between 17- and 18-positions are double bonds respectively. These PGs have a double bond between 13- and 14-position and a hydroxyl group on 15-position.

PGs exhibit various kinds of pharmacological and physiological activity. For instance, it has been known that $PGE_1$s and $PGE_2$s have a tracheobronchodilation activity but also have side effects such as enterocontraction, intraocular hypertension, and especially stimuli against bronchi.

Human and animal metabolites have also been found to contain PGEs in which the bond between 13- and 14-positions is a single bond (saturated) and the carbon atom of 15-position forms a carbonyl group; or in which the bond between 13- and 14-positions is a double bond and the carbon atom of 15-position constitutes carbonyl group.

15-keto-prostaglandin E (noted as 15-keto-PGE hereinafter) and 13,14-dihydro-15-keto-prostaglandin E (noted as 13,14-dihydro-15-keto-PGE hereinafter) are substances naturally produced by an enzyme in the metabolism of prostaglandin E (noted as PGE hereinafter) in a living body. These 15-keto-PGE have been considered to be physiologically and pharmacologically inactive substances (Acta Physiologica Scandinavica, vol. 66, pp509 (1966)). It has never been recognized that these 15-keto-PGE have tracheobronchodilation activity.

US-A-4 038 308 discloses 11-deoxy PGE derivatives which exert bronchodilating effects.

It has been found that some kinds of metabolites of PGs exhibit activity of tracheaectasy and/or bronchoectasy (referred to as tracheobronchodilation hereinafter).

The present invention provides the use of a composition comprising a 15-keto-PGE having at least one substituent at any of the 3-, 6-, 16-, 17-, 19- and 20- positions and a physiologically acceptable carrier for the manufacture of a medicament effective for tracheobronchodilation.

In the present specification 15-keto-PGEs are named according to the following nomenclature. The 15-keto-PGEs have a following basic structure:

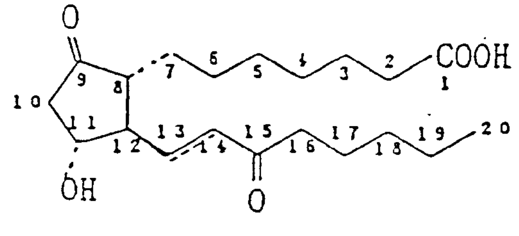

( α-chain)

( ω-chain)

and the position number of carbon atom constituting α-chain, ω-chain and five members ring in the prostaglandin skeleton structure is used as it is in the nomenclature. That is, the numbering of the carbon atoms in the skeleton structure starts with the carbon atom constituting carboxylic acid at the terminal position of the α-chain and continues through the five membered ring to the ω-chain i.e. 1 to 7 are the carbon atoms in the

α-chain, 8 - 12 are the carbon atoms in the five membered ring, and 13 - 20 are the carbon atoms in the ω-chain. In a compound having an α-chain with less than 7 carbon atoms, the position number is simply eliminated in order from 2 to 7 without any change of the position number of the other carbons. In other words, 15-keto-PGEs having 6 carbon atoms in the α-chain have no 2-position, i.e. 15-keto-PGEs of such compound are not renamed as 14-keto-PGEs. Of there are more than M carbon atoms in the α-chain, the "extra" carbon atoms in the chain are named as a substituent at the 2-position without any change of the position number of the other carbons. Therefore, 15-keto-PGEs having 8 carbon atoms in the α-chain are named as 15-keto-2-dicarboxy-2-acetic acid-PGEs. If the number of carbon atoms in the ω-chain dereases, the position number is named as reducing it from the carbon of position number 20 (referred to as 20-position hereinafter, and similarly referred to other positions) one by one. If the number of the carbon atoms in the ω-chain is greater than 20, the "increase" is named as a substituent at the 20-position. That is, 15-keto-PGEs having 10 carbon atoms in the ω-chain are named as 15-keto-20-ethyl-PGEs.

The above formula expresses a specific configuration which is most typical one, and in this specification compounds having such a configuration are expressed without any further descriptions.

PGEs have a hydroxy group on the carbon atom of 11-position in general, but in the present specification the term "PGEs" includes prostaglandins having other groups at the 11-position instead of the hydroxyl group of normal PGE. Such PGEs are named as 11-dehydroxy-11-substituent-PGEs, for instance, 11-dehydroxy-11-methyl-PGEs in case of the substituent being a methyl group.

PGEs are classified to $PGE_1$ and $PGE_2$ according to the bonds between 5- and 6-positions.

$PGE_1$ and its derivatives (referred to as $PGE_1$s hereinafter) are nominated to a group of compounds in which the bonds between 5- and 6-positions is a single bond. $PGE_2$ and its derivatives (referred to as $PGE_2$s hereinafter) belong to a group of compounds in which the bond between 5- and 6-positions is a cis-double bond. Therefore, PGEs having a structure of

$$\underset{7}{-CH_2}-\underset{6}{C(O)}-\underset{5}{CH_2}-$$

are named as 6-keto-$PGE_1$s, and PGEs having a structure of

$$\underset{7}{-CH_2}-\underset{6}{C}\equiv\underset{5}{C}-$$

are named as 5,6-dehydro-$PGE_2$s.

The substituted 15-keto-PGEs used in the present invention include for example substituted

13,14-dihydro-15-keto-$PGE_1$s and their derivatives,
13,14-dihydro-15-keto-$PGE_2$s and their derivatives,
13,14-dihydro-15-keto-$PGE_3$s and their derivatives,
15-keto-$PGE_1$s and their derivatives,
15-keto-$PGE_2$s and their derivatives,
15-keto-$PGE_3$s and their derivatives.

The tracheobroncholidation is remarkably expressed in 15-keto-PGEs esters represented by the following basic structural formula:

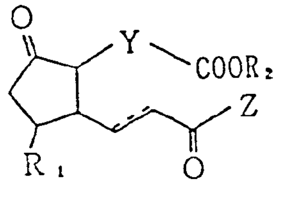

(I)

wherein $R_1$ is a hydroxyl group, a hydroxyalkyl group, or an alkyl group; Y is a saturated or unsaturted hydrocrbon moiety having 2 - 6 carbon atoms wherein a portion of carbon atoms constituting the hydrocarbon moiety may be carbonyl or a portion of hydrogen atoms constituting the hydrocarbon moiety may be substituted with other atoms or groups; Z is a saturated or an unsaturated hydrocarbon moiety which may constitute a straight chain or a ring, wherein a portion of hydrogen atoms of the hydrocarbon moiety may be substituted with one or more other atom(s) or group(s); $R_2$ is a hydrogen atom, a physiologically acceptable salt or ester residue;

Subject to the proviso that the compound of formula (I) has at least one substituent at one of the 3-, 6-, 16-, 17-, 19-, or 20-positions

Y which represents a saturated or an unsaturated hydrocarbon moiety having 2 - 6 carbon atoms includes an aliphatic hydrocarbon such as an alkylene group, an alkenylene group, and an alkynylene group. Y may preferably be a hydrocarbon chain having 6 carbon atoms.

Examples of PGEs of which Y is an unsaturated hydrocarbon moiety are $PGE_2$s, 5,6-dehydro-$PGE_2$s, and PGEs of which the bond between 2- and 3-positions is unsaturated.

Y may include a carbonyl group. A typical example of such a compound is a 6-keto-$PGE_1$.

The hydrocarbon moiety represented by Y may be substituted with one or more other atom(s) or group(s), for example, halogen atoms such as a fluorine atom, a chlorine atom, typically a fluorine atom; an alkyl group such as methyl, ethyl and a hydroxyl group. Typical examples of such substituents are 15-keto-PGEs having one or more alkyl group(s) on the carbon atom of 6 position.

Z represents a saturated or an unsaturated hydrocarbon moiety having 1 - 10 carbon atoms. The hydrocarbon moiety may be an aliphatic hydrocarbon or a cyclic hydrocarbon itself or in part. The hydrocarbon moiety represented by Z may be substituted with one or more other atom(s) or group(s).

The number of the carbon atoms of Z is preferably 3 - 7 in the straight chain. PGEs in which carbon numbers of Z are 5 are typical PGs. Therefore, the PGEs in which carbon numbers of the hydrocarbon moiety represented by Z are 6 or more than 6 are nominated as PGEs having a substituent on the carbon atom at the 20-position. That is, PGEs in which the number of carbon atoms of Z is 6 are nominated as 20-methyl-PGEs.

Though the hydrocarbon moiety represented by Z may have one or more unsaturated bond(s) at any position, a saturated hydrocarbon is more preferable. Examples of the hydrocarbon moiety having a cyclic ring are a cyclopentyl or a cyclohexyl containing a carbon atom itself of the 16- or 17-position as a ring constituting member.

The hydrocarbon moiety represented by Z may be substituted with one or more other atom(s) or group(s), for example, a halogen atom such as a fluorine atom or a chlorine atom; an alkyl group such as methyl, ethyl, isopropyl, isopropenyl; an alkoxy group such as methoxy, ethoxy; a hydroxyl group; a phenyl group; and a phenoxy group The substituent(s) may be preferably located at the 16-, 17-, 19- and/or 20-position, but not restricted. Examples of preferable compounds include those having one or two, different or identical atom(s) and/or groups, for example, a halogen atom such as a fluorine atom; an alkyl group such as a methyl, ethyl group; an aromatic group such as phenyl, benzyl, phenoxy, which may have substituents, or hydroxyl or other group at the 16-position. Other examples of preferable compounds include those having a cycloalkyl group such as a cyclopentyl or cyclohexyl group which contains the carbon atom at the 16-position as a constituent of the cyclic ring; an alkyl group such as methyl, and ethyl at the 17- or 19-position; an alkyl group such as methyl, ethyl, isopropyl, and isopropenyl; an alkoxy group such as methoxy, ethoxy, propoxy and the like at the 20-position.

A generic name of PGEs is used to compounds having a prostanoic acid structure in which the carbon atom at the 11-position has a hydroxyl group, and the carbon atom at the 9-position forms a carbonyl group. In the present specification a prostanoic acid compound in which the hydroxyl group at the 11-position is substituted with a hydroxyalkyl group or an alkyl group is also named as a PGE. Therefore, the 15-keto-PGEs of the present invention include compounds in which $R_1$ of the general formula (I) represents a hydroxyalkyl group or an alkyl group, for example, as a hydroxyalkyl group hydroxymethyl, 1-hydroxyethyl, 2-hydroxyethyl and 1-methyl-1-hydroxyethyl groups; as an alkyl group, methyl and ethyl groups are preferably exemplified.

The steric configuration of $R_1$ with respect to the carbon of 11-position may be $\alpha$, $\beta$ or a mixture thereof.

Furthermore, examples of the PGEs included in the present invention are 13,14-dihydro compounds which are saturated at the 13- and 14-positions, and rather more preferable effects can be achieved from such 13,14-dihydro compounds.

The 15-keto-PGEs used in the present invention may include various kinds of isomers such as tautomeric isomers, otpical isomers, and geometric isomers. As an example of such isomers there is exemplified a tautomeric isomer between the hydroxyl group at the 11-position and the carbonyl group at the 15-position of 15-keto-PGEs. The latter tautomeric isomer is liable to be caused in 15-keto-PGEs having an electron withdrawing group such as fluorine atom at the 16-position.

A hemiacetal, a tautomeric isomer between the hydroxyl group at the 11-position and the carbonyl group at the 15-position, may be sometimes formed, and an equilibrium mixture of the compound of R being a hydroxyl group and a hemiacetal may be given. Such an equilibrium mixture or a tautomeric isomer is also included in the 15-keto-PGEs of the present invention.

An equilibrium mixture of the isomers such as racemic or equilibrium mixture between a hydroxyl group and a hemiacetal in tautomerism also exhibits similar effects to a single compound.

The most preferable 15-keto-PGEs according to the present invention are PGEs in which the 13- and 14-positions are saturated and the carbon atom of 15-position constitutes a carbonyl group. The corresponding 15 - keto - PGES in which there is an unsaturated bond between the 13 - and 14 - positions are however also

of interest.

As 15-keto-PGEs exhibit strong tracheobronchodilation activity, these 15-keto-PGEs are expected to be tracheobronchodilators, and due to such an activity they can be used as bronchial asthematics or medicines for patients who suffer from the increase of airway resistance.

The 15-keto-PGEs of the present invention may be the free acid, a physiologically acceptable salt or an ester thereof.

As a salt there are exemplified alkaline metal salts such as sodium salt, and potassium salt alkaline earth metal salts such as calcium salt, and magnesium salt; ammonium salts; physiologically acceptable amine salts such as monomethylamine salt, dimethylamine salt, cyclopentylamine salt, benzylamine salt, piperidine salt, tromethamine salt, monoethanolamine salt, diethanolamine salt, monomethylmonoethanolamine salt, lysine salt, and tetraalkylammonium salt.

Useful esters ($R_2$) of 15-keto-PGEs may include a saturated or an unsaturated lower alkyl ester which may have a branched chain such as methyl, ethyl, propyl, n-butyl, isopropyl, t-butyl, 2-ethylhexyl, and allyl. An aliphatic cyclic ester such as cyclopropyl, cyclopentyl, and cyclohexyl; an aromatic ester such as benzyl, and phenyl, which may have substituents; a hydroxyalkyl or an alkoxyalkyl ester such as hydroxyethyl, hydroxyisopropyl, hydroxypropyl, polyhydroxyethyl, polyhydroxyisopropyl, methoxyethyl, ethoxyethyl, and methoxyisopropyl; a trialkylsilyl ester such as trimethylsilyl, and triethylsilyl.; a heterocyclic ester such as tetrahydropyranyl. Preferable esters for the present invention are a lower alkyl ester which may have a branched chain, for instance, methyl, ethyl, propyl, n-butyl, isopropyl, t-butyl; a benzyl ester; a hydroxyalkyl ester such as hydroxyethyl, and hydroxy isopropyl.

The activity of the tracheobronchodilation can be exhibited in any 15-keto-PGEs in which the carboxyl group of $\alpha$-chain is free, esterified or salts forms, but particularly strongly exhibited in the compounds of which the bond between the 2- and 3-positions is a double bond, or the bond between the 5- and 6-positions is a triple bond.

Preferable substituents which may be attached to one or more carbon atom(s) at the 3-, 17- and/or 19-position are $C_1$- $C_4$ alkyl groups, especially methyl or ethyl. Preferable substituents at the 16-position may be, for instance, one or more lower alkyl group(s) such as methyl or ethyl group, hydroxyl group(s), halogen atom(s) such as a chlorine atom or a fluorine atom. Preferable substituent(s) on 20-position may be, for instance, saturated or unsaturated alkyl group(s), especially $C_1$ - $C_4$ alkyl group(s), $C_1$ - $C_4$ alkyl group(s) having alkoxy group(s) or alkoxy substituent(s).

The carbon atom at the 6-position may constitute a carbonyl group, and the configuration with respect to the carbon atom of 11-position may be $\alpha$, $\beta$ or mixture thereof.

Compounds having one or two lower alkyl group(s) such as methyl, and ethyl are prefered preferable because of its high activity of tracheobronchodilation.

Further, compounds having a shorter $\omega$-chain (compounds in which the number of carbon atoms of $\omega$-chain is less than 8) and an alkoxy group, a phenoxy group, a phenyl group or others on the terminal position of the $\omega$-chain are also useful.

The PGEs of the present invention may include any isomers of the above, for instance, tautomers between the hydroxyl group at 11-position and the carbonyl group at 15-position, optical isomers, or geometric isomers.

The tautomers between the hydroxyl group at 11-position and the carbonyl group at the 15-position can be more easily formed when 15-keto-PGEs have an electron withdrawing group such as a fluorine atom at the 16-position.

The equilibrium mixture of isomers, for instance, racemic mixture, tautomers of hydroxyl compounds and hemiacetals, also exhibits an activity similar to a single (non isomeric) compound thereof.

Examples of the typical compounds of the present invention are:

15-keto-PGE and 13,14-keto-dihydro-15-keto-PGE and their derivatives such as 6-keto-derivatives, $\Delta^2$-derivatives, 3R,S-methyl-derivatives, 16R,S-methyl-derivatives, 16,16-dimethyl-derivatives, 16R,S-fluoro-derivatives, 16,16-difluoro-derivatives, 17S-methyl-derivatives, 19-methyl-derivatives, and 20-methyl-derivatives.

In the present specification PGEs are named based on a prostanoic acid skeleton, but they can be named according to IUPAC nomenclature, according to which, for instance, $PGE_1$, is nominated as 7-{(1R,2R,3R)-3-hydroxy-2-[(E)-(3S)-3-hydroxy-1-octenyl]-5-oxo-cyclopentyl}-heptanoic acid; $PGE_2$ is nominated as (z)-7-{(1R,2R,3R)-3-hydroxy-2-[(E)-(3S)-3-hydroxy-1-octenyl]-5-oxo-cyclopentyl}-hept-5-enoic acid; 13,14-dihydro-15-keto-16R,S-fluoro-$PGE_2$ is nominated as (z)-7-{(1R,2R,3R)-3-hydroxy-2-[(4R,S)-4-fluoro-3-oxo-1-octyl]-5-oxo-cyclopentyl}-hept-5-enoic acid; 13,14-dihydro-15-keto-20-ethyl-11-dehydroxy-11R-methyl $PGE_2$ methyl ester is nominated as methyl 7-{(1R,2S,3R)-3-methyl-2-[3-oxo-1-decyl]-5-oxo-cyclopentyl}-hept-5-enoate; and 13,14-dihydro-6,15-diketo-19-methyl-$PGE_1$ ethyl ester is nominated as ethyl 7-{(1R,2R,3R)-3-hydroxy-2-(7-methyl-3-oxo-1-octyl)-5-oxocyclopentyl}-6-oxo-heptanoate.

The 15-keto-PGEs used in this invention may be prepared, for example, by the method given in Japanese Patent Application No. 18326/1988.

A practical preparation of the 13,14-dihydro-15-keto PGEs involves the following steps; as shown in the synthetic charts (I) to (III), reaction of the aldehyde (2) prepared by the Collins oxidation of commercially available (-)-Corey lactone (1) with dimethyl (2-oxoheptyl)phosphate anion to give α,ß-unsaturated ketone (3), reaction of the α,β-unsaturated ketone (3) to the corresponding saturated ketone (4), protection of the carbonyl group of the ketone (4) with a diol to the corresponding ketal (5), and deprotection of the p-phenylbenzoyl group to give the corresponding alcohol (6) followed by protection of the newly derived hydroxy group with dihydropyrane to give the corresponding tetrahydropyranyl ether (7). According to the above process, a precursor of PGEs in which the ω-chain is a 13,14-dihydro-15-keto-alkyl group is prepared.

Using the above tetrahydropyranyl ether (7), 6-keto-$PGE_1$s (15) of which a group constituted with carbon atoms of 5-, 6- and 7-positions is

$$-\underset{7}{CH_2}-\underset{6}{C(O)}-\underset{5}{CH_2}-,$$

may be prepared in the following steps; reduction of the tetrahydropyranyl ether (7) with, for example, diisobutyl aluminum hydride to give the corresponding lactol (8), reaction of the lactol (8), with the ylide generated from (4-carboxybutyl)triphenyl phosphonium bromide followed by esterification (10), cyclization between the 5,6-double bond and the hydroxyl group at 9-position with NBS or iodine to give the halogenated compound (11), dehydrohalogenation of the compound (11) with, for example, DBU to give the 6-keto compound (13) followed by Jones oxidation and removal of the protecting groups.

Furthermore, $PGE_2$s (19) of which a group constituted with carbon atoms of 5-, 6- and 7-position is

$$-\underset{7}{CH_2}-\underset{6}{CH}=\underset{5}{CH}-$$

may be prepared in the following steps; as shown in the synthetic chart II, reduction of the above the tetrahydropyranyl ether (7) to give the lactol (8), reaction of the resultant lactol (8) with the ylide derived from (4-carboxybutyl)triphenyl phosphonium bromide to give the carboxylic acid (16) followed by esterification to give ester (17), Jones oxidation of the esters (17) to give the compound (18), and removal of the protecting groups.

Using the above the tetrahydropyranyl ether (7) as starting material, the compound having

$$-\underset{7}{CH_2}-\underset{6}{CH_2}-\underset{5}{CH_2}-$$

may be prepared by using the same process for preparing $PGE_2$s having

$$-\underset{7}{CH_2}-\underset{6}{CH}=\underset{5}{CH}-$$

and applying the resultant compound (18) to catalytic reduction for reducing the double bond between the 5- and 6-positions followed by removal of the protection groups.

Synthesis of 5,6-dehydro-$PGE_2$s having

$$-\underset{7}{CH_2}-\underset{6}{C}\equiv\underset{5}{C}-$$

may be carried out by alkylation of the resulting a copper enolate derived after 1,4-addition of a monoalkylcopper complex or a dialkylcopper complex of the following formula:

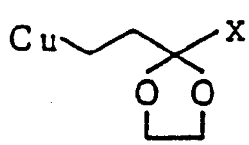 , 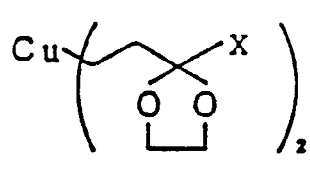

to 4R-t-butyldimethylsilyloxy-2-cyclopenten-1-one with 6-alkoxycarbonyl-1-iodo-2-hexyne or derivatives.

The 11-β type PGEs can be prepared according to the synthetic chart III.

11-Dehydroxy-11-methyl-PGEs were obtained by reacting PGAs obtained by Jones oxidation of the hydroxy group at the C-9 position of the 11-tosylate with a dimethyl copper complex. Alternatively, PGAs may

be prepared by converting an alcohol obtained after elimination of p-phenylbenzoyl group to a tosylate; treating the tosylate with DBU to give an unsaturated lactone, which is then converted to a lactol; introducing an α-chain using Wittig reaction; and oxidizing the resulting alcohol (C-9 position).

11-Dehydroxy-11-hydroxymethyl-PGE can be obtained by a benzophenone-sensitized photoaddition of methanol to PGA.

The 15-keto-PGEs of this invention may be used as a medicine for animals and human beings and usually applied systemically or locally by the method of oral administration, oral administration by spraying, intravenous injection (including instillation), subcutaneous injection, suppository, spraying, coating and, gargling. Dose is determined depending on the animal to be treated, the human patient, age, body weight, symptom, therapeutic effect, administration route and, treating time, but is preferably 0.001 - 500 mg/kg.

As solid composition of this invention for oral administration, tablets, troches, capsules, pills, powders and granules are included. The solid composition containing one or more active substances is mixed with at least one inactive diluent such as lactose, mannitol, glucose, hydroxypropyl cellulose, fine crystalline cellulose, starch, polyvinyl pyrrolidone, and magnesium aluminate metasilicate. The composition may contain additives other than the inactive diluent, such as lubricants (e.g. magnesium stearate), a disintegrator (e.g. cellulose calcium gluconates), stabilizers (e.g. α-, β- or γ-cyclodextrins, etherated cyclodextrins (e.g. dimethyl-α-, dimethyl-β-, trimethyl-β- or hydroxypropyl-β-cyclodextrins), branched cyclodextrins (e.g. glucosyl- or maltosyl-cyclodextrins), formyl cyclodextrins, sulphur-containing cyclodextrins, misoprotols or phospholipids. Such cyclodextrins may form an inclusion compound with 15-keto-PGEs in some cases to increase the stability of the compounds. The stability may be often increased by forming a lyposome with phospholipids. Tablets and pills may be coated with an enteric or gastroenteric film such as white sugar, gelatin, hydroxypropylcellulose and hydroxypropylmethylcellulose phthalates, if necessary, and furthermore they may be covered with two or more layers. Additionally, the composition may be in the form of capsules made of substance easily absorbed such as gelatin.

Liquid compositions for oral administration include pharmaceutically acceptable emulsions, solutions, suspensions, syrups, and elixirs, and contain a generally used inactive diluent such as purified water or ethyl alcohol. The composition may contain additives such as wetting agents, suspending agents, adhesives (e.g. saponins), sweeteners, flavours, perfumes and preservatives.

The compositions for oral administration may contain one or more active substance.

The composition of the present invention may be sprays which can be prepared according to well known methods. The sprays are particularly suitable for the prevention or treatment of paroxysm of asthema, which may be prepared, for instance, by dispersing effective compounds with the aid of surface active agent into blowing agents such as Flon™.

The invention of this invention for non-oral administration includes sterile aqueous or nonaqueous solutions, suspensions, and emulsions. Diluents for the aqueous solution or suspension include, for example, distilled water for injection, physiological saline and Ringer's solution. Diluents for the nonaqueous solution and suspension include, for example, propylene glycol, polyethylene glycol, vegetable oils such as olive oil, and alcohols such as ethanol and polysorbates. The composition may contain other additives such as preservatives, wetting agents, emulsifying agents, and dispersing agents. These are sterilized by filtration through, e.g. a bacteria-retaining filter, compounding with a sterilizer, gas sterilization or radiation sterilization. These can be prepared by producing a sterilized water or a sterilized solvent for injection before use.

Further, in case that the drugs must be rapidly absorbed such as in asthma the tracheobronchodilator of the present invention may be formulated to a sublingual tablet, which may be prepared by mixing or dissolving the effective components with carriers such as glycerin, lactose, mannitol, and sorbitol.

The tracheobronchodilator of the present invention may be formulated to a collutorium.

The 15-keto-PGEs of the present invention are effective to diseases with increase of airway resistance such as bronchial asthmatics, infectious asthmatic bronchitis, and chronic bronchitis.

Example 1 (tracheaectasy)

A male guinea pig weighing about 300 g was sacrified and immediately the blood was drawn by cutting the arteriae femoral. A trachea was picked out and cut lengthwise at the opposite side of tracheal smooth muscle and cut into circular slices between cartilages. The obtained seven circular slices of the trachea were tied like as a chain with a thread, and hung into Magnus tube.

After the circular slices of trachea were held for 60 - 90 minutes until it became stable (disappearance of convulsion), they were administered with 5.4 x $10^{-4}$ M of histamine to be contracted. After the contraction reached to maximum, each test drug was cumulatively administered so as to inhibit the contraction caused by the histamine. The concentration of administered drug exhibiting the inhibition rates of 20 % and 50 % are

represented by $IC_{20}$ and $IC_{50}$ respectively. The results are shown in Table 1.

Table 1

| Test drugs | $IC_{20}$ (M) | $IC_{50}$ (M) |
|---|---|---|
| 1 | $8 \times 10^{-7}$ | $7 \times 10^{-6}$ |
| 2 | $2 \times 10^{-6}$ | $6 \times 10^{-6}$ |
| 3 | $9 \times 10^{-6}$ | - |
| 4 | $6 \times 10^{-6}$ | - |
| 5 | $4 \times 10^{-6}$ | $1 \times 10^{-5}$ |
| 6 | $1 \times 10^{-6}$ | $5 \times 10^{-6}$ |
| 7 | $3 \times 10^{-6}$ | - |
| 8 | $5 \times 10^{-6}$ | $2 \times 10^{-5}$ |
| 9 | $1 \times 10^{-7}$ | $4 \times 10^{-7}$ |

| Test drugs | $IC_{20}$ (M) | $IC_{50}$ (M) |
|---|---|---|
| 10 | $1 \times 10^{-7}$ | $6 \times 10^{-7}$ |
| 11 | $8 \times 10^{-7}$ | $4 \times 10^{-6}$ |
| 12 | $6 \times 10^{-7}$ | $3 \times 10^{-6}$ |
| 13 | $1 \times 10^{-6}$ | $3 \times 10^{-5}$ |
| 14 | $9 \times 10^{-7}$ | $3 \times 10^{-5}$ |
| 15 | $5 \times 10^{-7}$ | $4 \times 10^{-6}$ |
| 16 | $5 \times 10^{-6}$ | $4 \times 10^{-5}$ |
| 17 | $3 \times 10^{-6}$ | $1 \times 10^{-5}$ |
| 18 | $1 \times 10^{-6}$ | $1 \times 10^{-5}$ |
| 19 | $1 \times 10^{-6}$ | $5 \times 10^{-6}$ |
| 20 | $6 \times 10^{-7}$ | $2 \times 10^{-6}$ |
| 21 | $4 \times 10^{-7}$ | $2 \times 10^{-6}$ |
| 22 | $2 \times 10^{-6}$ | $8 \times 10^{-6}$ |
| 23 | $5 \times 10^{-7}$ | $2 \times 10^{-6}$ |
| 24 | $2 \times 10^{-6}$ | $1 \times 10^{-5}$ |
| 25 | $4 \times 10^{-5}$ | - |
| 26 | $1 \times 10^{-6}$ | $7 \times 10^{-6}$ |
| 27 | $8 \times 10^{-8}$ | $4 \times 10^{-7}$ |
| 28 | $7 \times 10^{-7}$ | $7 \times 10^{-6}$ |
| 29 | $6 \times 10^{-7}$ | $7 \times 10^{-6}$ |
| 30 | $6 \times 10^{-7}$ | $2 \times 10^{-6}$ |
| 31 | $4 \times 10^{-7}$ | $2 \times 10^{-6}$ |
| 32 | $1 \times 10^{-6}$ | $3 \times 10^{-5}$ |
| 33 | $1 \times 10^{-6}$ | $7 \times 10^{-6}$ |
| 34 | $3 \times 10^{-6}$ | $1 \times 10^{-5}$ |

| Test drugs | $IC_{20}$ (M) | $IC_{50}$ (M) |
|---|---|---|
| 35 | $9 \times 10^{-7}$ | $2 \times 10^{-5}$ |
| 36 | $5 \times 10^{-7}$ | $5 \times 10^{-6}$ |
| 37 | $4 \times 10^{-7}$ | $8 \times 10^{-6}$ |
| 38 | $6 \times 10^{-7}$ | $7 \times 10^{-6}$ |
| 39 | $3 \times 10^{-6}$ | $6 \times 10^{-5}$ |
| 40 | $2 \times 10^{-6}$ | $4 \times 10^{-5}$ |
| 41 | $4 \times 10^{-6}$ | - |
| 42 | $1 \times 10^{-5}$ | - |
| 43 | $1 \times 10^{-5}$ | - |
| 44 | $1 \times 10^{-5}$ | - |
| 45 | $2 \times 10^{-6}$ | $1 \times 10^{-5}$ |
| 46 | $1 \times 10^{-5}$ | - |
| 47 | $6 \times 10^{-7}$ | $3 \times 10^{-6}$ |
| 48 | $1 \times 10^{-5}$ | - |
| 49 | $7 \times 10^{-6}$ | - |
| 50 | $2 \times 10^{-5}$ | - |
| 51 | $1 \times 10^{-6}$ | $1 \times 10^{-5}$ |
| 52 | $1 \times 10^{-6}$ | - |
| 53 | $1 \times 10^{-5}$ | - |
| 54 | $8 \times 10^{-6}$ | $3 \times 10^{-5}$ |
| 55 | $2 \times 10^{-6}$ | $1 \times 10^{-5}$ |
| 56 | $2 \times 10^{-9}$ | $8 \times 10^{-8}$ |
| 57 | $1 \times 10^{-8}$ | $1 \times 10^{-7}$ |
| 58 | $1 \times 10^{-6}$ | $3 \times 10^{-5}$ |
| 59 | $1 \times 10^{-6}$ | $4 \times 10^{-6}$ |

| Test drugs | $IC_{20}$ (M) | $IC_{50}$ (M) |
|---|---|---|
| 60 | $3 \times 10^{-6}$ | $7 \times 10^{-6}$ |
| 61 | $7 \times 10^{-7}$ | $2.5 \times 10^{-6}$ |
| 62 | $4 \times 10^{-6}$ | - |
| 63 | $1 \times 10^{-5}$ | - |
| 64 | $3 \times 10^{-7}$ | $1 \times 10^{-6}$ |

Test Drugs

1. 13,14-dihydro-15-keto-$PGE_1$
2. 13,14-dihydro-15-keto-$PGE_1$ ethyl ester
3. 13,14-dihydro-15-keto-$\Delta^2$-$PGE_1$
4. 13,14-dihydro-15-keto-$\Delta^2$-$PGE_1$ methyl ester
5. 13,14-dihydro-6,15-diketo-$PGE_1$ methyl ester
6. 13,14-dihydro-6,15-diketo-$PGE_1$ ethyl ester
7. (±)13,14-dihydro-6,15-diketo-$PGE_1$ ethyl ester
8. 13,14-dihydro-6,15-diketo-$PGE_1$ n-butyl ester
9. 13,14-dihydro-6,15-diketo-16R,S-methyl-$PGE_1$ methyl ester
10. 13,14-dihydro-6,15-diketo-16R,S-methyl-$PGE_1$ ethyl ester
11. 13,14-dihydro-6,15-diketo-16,16-dimethyl-$PGE_1$ ethyl ester
12. 13,14-dihydro-6,15-diketo-16R,S-fluoro-$PGE_1$ ethyl ester
13. 13,14-dihydro-6,15-diketo-19-methyl-$PGE_1$ methyl ester
14. 13,14-dihydro-6,15-diketo-19-methyl-$PGE_1$ ethyl ester
15. 13,14-dihydro-6,15-diketo-20-methyl-$PGE_1$ ethyl ester
16. 13,14-dihydro-15-keto-$PGE_2$
17. 13,14-dihydro-15-keto-$PGE_2$ methyl ester
18. 13,14-dihydro-15-keto-$PGE_2$ ethyl ester
19. 13,14-dihydro-15-keto-$PGE_2$ n-propyl ester
20. 13,14-dihydro-15-keto-$PGE_2$ n-butyl ester
21. 13,14-dihydro-15-keto-$PGE_2$ benzyl ester
22. 13,14-dihydro-15-keto-$PGE_2$ hydroxyethyl ester
23. 13,14-dihydro-15-keto-$PGE_2$ isopropyl ester
24. 13,14-dihydro-15-keto-$\Delta^2$-$PGE_2$ methyl ester
25. 13,14-dihydro-15-keto-3R,S-methyl-$PGE_2$ methyl ester
26. 13,14-dihydro-15-keto-3R,S-methyl-$PGE_2$ ethyl ester
27. 13,14-dihydro-15-keto-16R,S-methyl-$PGE_2$ methyl ester
28. 13,14-dihydro-15-keto-16R,S-methyl-$PGE_2$ ethyl ester
29. 13,14-dihydro-15-keto-3R,S-16R,S-dimethyl-$PGE_2$ methyl ester
30. 13,14-dihydro-15-keto-16,16-dimethyl-$PGE_2$ methyl ester
31. 13,14-dihydro-15-keto-16,16-dimethyl-$PGE_2$ ethyl ester
32. 13,14-dihydro-15-keto-16R,S-hydroxy-$PGE_2$ ethyl ester
33. 13,14-dihydro-15-keto-16R,S-fluoro-$PGE_2$
34. 13,14-dihydro-15-keto-16R,S-fluoro-$PGE_2$ methyl ester
35. 13,14-dihydro-15-keto-16R,S-fluoro-$PGE_2$ ethyl ester
36. 13,14-dihydro-15-keto-16R,S-fluoro-20-methyl-$PGE_2$ methyl ester
37. 13,14-dihydro-15-keto-16R,S-fluoro-11-dehydroxy-11R-methyl-$PGE_2$ ethyl ester
38. 13,14-dihydro-15-keto-11-dehydroxy-11R-methyl-$PGE_2$ ethyl ester
39. 13,14-dihydro-15-keto-17S-methyl-$PGE_2$ methyl ester
40. 13,14-dihydro-15-keto-19-methyl-$PGE_2$ methyl ester
41. 13,14-dihydro-15-keto-19-methyl-$PGE_2$ ethyl ester

42. 13,14-dihydro-15-keto-20-methoxy-$PGE_2$ methyl ester
43. 13,14-dihydro-15-keto-20-methoxy-$\Delta^2$-$PGE_2$ methyl ester
44. 13,14-dihydro-15-keto-3R,S-methyl-20-methoxy-$PGE_2$ methyl ester
45. 13,14-dihydro-15-keto-16,16-dimethyl-20-methoxy-$PGE_2$ methyl ester
46. 13,14-dihydro-15-keto-5,6-dehydro-20-methoxy-$PGE_2$ methyl ester
47. 13,14-dihydro-15-keto-18-methoxy-19,20-dinol-$PGE_2$ methyl ester
48. 13,14-dihydro-15-keto-20-isopropylidene-$PGE_2$
49. 13,14-dihydro-15-keto-20-isopropylidene-$PGE_2$ methyl ester
50. 13,14-dihydro-15-keto-20-ethyl-$PGE_2$ methyl ester
51. 13,14-dihydro-15-keto-20-ethyl-$PGE_2$ ethyl ester
52. 13,14-dihydro-15-keto-20-ethyl-11-dehydroxy-11R-methyl-$PGE_2$ methyl ester
53. 13,14-dihydro-15-keto-20-n-propyl-$PGE_2$ methyl ester
54. 15-keto-$PGE_2$
55. 15-keto-16R,S-fluoro-$PGE_2$ methyl ester
56. $PGE_1$
57. $PGE_2$
58. 13,14-dihydro-15-keto-17S-methyl-$PGE_2$ ethyl ester
59. 13,14-dihydro-15-keto-20-methyl-$PGE_1$
60. 15-keto-16R,S-fluoro-$PGE_2$
61. 15-keto-17S-methyl-$PGE_2$ ethyl ester
62. 13,14-dihydro-15-keto-16R,S-fluoro-$PGE_1$
63. 13,14-dihydro-6,15-diketo-18-methyl-$PGE_1$ ethyl ester
64. 13,14-dihydro-15-keto-16R,S-methyl-$PGE_1$ methyl ester

Example 2 (stimuli of trachea)

The strength of stimuli to trachea of humans was evaluated by actual spraying inhalation.

Each test drug was dissolved with a small amount of ethyl alcohol, to which distilled water was added to prepare a solution containing the test drug at a concentration of 10 μg/ml. Four panels (K, D, T and O) were sprayed into their throats with the each test solution at a rate of 2 ml/min using an ultrasonic inhaler (NE-U10B available from Omlon K.K.), and the strength of the stimuli was observed.

The results were shown in Table 2.

Table 2

| test sample | K | D | T | O |
|---|---|---|---|---|
| $PGE_1$ (comparative) | ++ | + | + | ± |
| 13,14-dihydro-6,15-diketo-16R,S-methyl-$PGE_1$ methyl ester | - | - | - | - |

- : no stimuli
± : slight stimuli
+ : strong stimuli
++: very strong stimuli

Example 3 (inhibition of increase of airway resistance)

A male guinea pig weighing 300 - 400 g was intraperitoneally anesthetized with urethane 1.5 g/kg. The test animal was cannulated through the trachea, and then bromopanchlonium was intravenously administered

at 0.3 mg/kg to make the test animal inactivate. The animal was respired using a vespirater for a small animal. The airway resistance was recorded on a recorder using a bronchospasin transducer. The administration of the test drug was made through a cannula into the jugular vein. The histamine was administered three times of each 3 µg/kg at 30 minute intervals, and the increase of the airway resistance was determined. After the determination the test drug was intervenously administered, and then 4th administration of histamin was done after one minute. The increase of airway resistance due to histamin before and after the administration of the test drug was determined, from which inhibition rate of the increase due to the test drug was evaluated. The results are shown in Table 4.

Table 4

| test sample | dose (µg/kg) | inhibition rate (%) |
|---|---|---|
| 13,14-dihydro-15-keto-16,16-difluoro-$PGE_2$ | 1<br>3 | 42<br>100 |
| $PGE_2$ | 3 | 92 |

## Claims

### Claims for the following Contracting States : AT, BE, CH, DE, FR, GB, IT, LI, LU, NL, SE

1. The use of a composition comprising a 15-keto-PGE having at least one substituent at any of the 3-, 6-, 16-, 17-, 19- and 20-positions and a physiologically acceptable carrier for the manufacture of a medicament effective for tracheobronchodilation.

2. The use according to the Claim 1, in which the 15-keto-PGE is represented by the following formula:

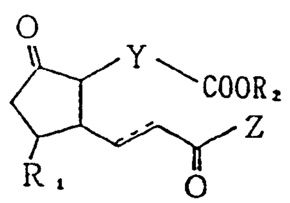

wherein $R_1$ is a hydroxyl group, a hydroxyalkyl group, or an alkyl group; Y is an optionally substituted, saturated or unsaturated hydrocarbon moiety having 2-6 carbon atoms at least one of which may be a carbonyl group; Z is an optionally substituted, saturated or an unsaturated hydrocarbon moiety constituting a straight chain having a carbon number of 1-10 or a ring; and $R_2$ is a hydrogen atom, a physiologically acceptable salt or ester residue.

3. The use according to Claim 1, in which the 15-keto-PGEs are 13,14-dihydro-15-keto-PGEs.

4. The use according to Claim 1, in which a carboxyl group in the terminal position of the α-chain of the 15-keto-PGE is esterified.

5. The use according to Claim 1, in which a bond between 2-position and 3-position of the 15-keto-PGE is a double bond.

6. The use according to Claim 1, in which the 15-keto-PGE possess a methyl group on 3-position.

7. The use according to Claim 1, in which the 15-keto-PGE is a 6,15-diketo-PGE.

8. The use according to Claim 1 in which the 15-keto-PGE is substituted at the 20-position with a $C_1$-$C_4$ alkyl group or alkoxy group.

9. The use according to Claim 1 in which the 15-keto-PGE is a 15-keto-16-mono or dihalogen-PGE.

10. The use according to Claim 9 wherein the 15-keto-PGE is a 15-keto-16-mono or difluoro-PGE.

11. The use according to Claim 1 in which the 15-keto-PGE is a 15-keto-16-mono or dialkyl-PGE.

12. The use according to Claim 11 in which the 15-keto-PGE is a 15-keto-16-mono or dimethyl-PGE.

13. The use according to Claim 1, in which the 15-keto-PGE is a 6,15-diketo- 16-alkyl-PGE.

14. The use according to Claim 1, in which the 15-keto-PGE is a 6,15-diketo-methyl-PGE.

15. The use according to Claim 1, in which the 15-keto-PGE is a 13,14-dihydro-6,15-diketo-16-alkyl-PGE.

16. The use according to Claim 1, in which the 15-keto-PGE is a 13,14-dihydro-6,15-diketo-16-methyl-PGE.

17. The use according to Claim 1 for the treatment of, or protection against, diseases caused by the increase of airway resistance.

18. The use according to Claim 17, in which the diseases are bronchial asthmatics, infectious asthmatic bronchitis or chronic bronchitis.

19. The use according to Claim 17, in which administration of the composition is by spraying, intravenous injection, subcutaneous injection, suppository coating or gargling.

**Claims for the following Contracting States : ES, GR**

1. A method for the manufacture of a composition effective for tracheobronchodilation, comprising admixing a 15-keto-PGE having at least one substituent at any of the 3-, 6-, 16-, 17-, 19- and 20-positions and a physiologically acceptable carrier of a medicament

2. A method according to the Claim 1, in which the 15-keto-PGE is represented by the following formula:

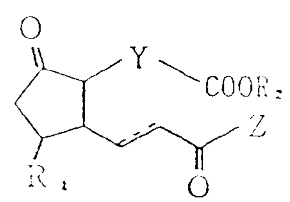

wherein $R_1$ is a hydroxyl group, a hydroxyalkyl group, or an alkyl group; Y is an optionally substituted, saturated or unsaturated hydrocarbon moiety having 2-6 carbon atoms at least one of which may be a carbonyl group; Z is an optionally substituted, saturated or an unsaturated hydrocarbon moiety constituting a straight chain having a carbon number of 1-10 or a ring; and $R_2$ is a hydrogen atom, a physiologically acceptable salt or ester residue.

3. A method according to Claim 1, in which the 15-keto-PGEs are 13,14-dihydro-15-keto-PGEs.

4. A method according to Claim 1, in which a carboxyl group in the terminal position of the α-chain of the 15-keto-PGE is esterified.

5. A method according to Claim 1, in which a bond between 2-position and 3-position of the 15-keto-PGE is a double bond.

6. A method according to Claim 1, in which the 15-keto-PGE possess a methyl group on 3-position.

7. A method according to Claim 1, in which the 15-keto-PGE is a 6,15-diketo-PGE.

8. A method according to Claim 1 in which the 15-keto-PGE is substituted at the 20-position with a $C_1$-$C_4$ alkyl group or alkoxy group.

9. A method according to Claim 1 in which the 15-keto-PGE is a 15-keto-16-mono or dihalogen-PGE.

10. A method according to Claim 9 wherein the 15-keto-PGE is a 15-keto-16-mono or difluoro-PGE.

11. A method according to Claim 1 in which the 15-keto-PGE is a 15-keto-16-mono or dialkyl-PGE.

12. A method according to Claim 11 in which the 15-keto-PGE is a 15-keto-16-mono or dimethyl-PGE.

13. A method according to Claim 1, in which the 15-keto-PGE is a 6,15-diketo-16-alkyl-PGE.

14. A method according to Claim 1, in which the 15-keto-PGE is a 6,15-diketo-methyl-PGE.

15. A method according to Claim 1, in which the 15-keto-PGE is a 13,14-dihydro-6,15-diketo-16-alkyl-PGE.

16. A method according to Claim 1, in which the 15-keto-PGE is a 13,14-dihydro-6,15-diketo-16-methyl-PGE.

17. A method according to Claim 1 wherein the medicament is for the treatment of, or protection against, diseases caused by the increase of airway resistance.

18. A method according to Claim 17, wherein the medicament is for the treatment of, or protection against, bronchial asthmatics, infectious asthmatic bronchitis or chronic bronchitis.

19. A method according to Claim 17, wherein the medicament is for administration by spraying, intravenous injection, subcutaneous injection, suppository coating or gargling.

## Patentansprüche

## Patentansprüche für folgende Vertragsstaaten : AT, BE, CH, DE, FR, GB, IT, LI, LU, NL, SE

1. Verwendung einer Zusammensetzung, die 15-Keto-PGE mit mindestens einem Substituenten an irgendeiner der 3-, 6-, 16-, 17-, 19- und 20-Stellungen und einen physiologisch annehmbaren Träger enthält, für die Herstellung eines Arzneimittels, das für die Tracheobronchodilatation wirksam ist.

2. Verwendung nach Anspruch 1, dadurch **gekennzeichnet**, daß das 15-Keto-PGE durch die folgende Formel:

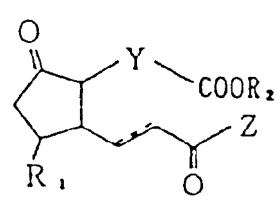

dargestellt wird, worin $R_1$ eine Hydroxylgruppe, eine Hydroxyalkylgruppe oder eine Alkylgruppe bedeutet; Y eine gegebenenfalls substituierte, gesättigte oder ungesättigte Kohlenwasserstoff-Gruppierung mit 2 bis 6 Kohlenstoffatomen, wovon mindestens eines eine Carbonylgruppe sein kann, bedeutet; Z eine gegebenenfalls substituierte, gesättigte oder ungesättigte Kohlenwasserstoff-Gruppierung, die eine gerade Kette mit einer Kohlenstoffzahl von 1 bis 10 oder ein Ring sein kann, bedeutet; und $R_2$ ein Wasserstoffatom, ein physiologisch annehmbares Salz oder einen Esterrest bedeutet.

3. Verwendung nach Anspruch 1, dadurch **gekennzeichnet**, daß die 15-Keto-PGEs 13,14-Dihydro-15-keto-PGEs sind.

4. Verwendung nach Anspruch 1, dadurch **gekennzeichnet**, daß die Carboxylgruppe in der Endstellung der α-Kette des 15-Keto-PGEs verestert ist.

5. Verwendung nach Anspruch 1, dadurch **gekennzeichnet**, daß die Bindung zwischen der 2-Stellung und der 3-Stellung des 15-Keto-PGEs eine Doppelbindung ist.

6. Verwendung nach Anspruch 1, dadurch **gekennzeichnet**, daß das 15-Keto-PGE eine Methylgruppe in der 3-Stellung besitzt.

7. Verwendung nach Anspruch 1, dadurch **gekennzeichnet**, daß das 15-Keto-PGE ein 6,15-Diketo-PGE ist.

8. Verwendung nach Anspruch 1, dadurch **gekennzeichnet**, daß das 15-Keto-PGE in der 20-Stellung mit einer $C_1$-$C_4$-Alkylgruppe oder Alkoxygruppe substituiert ist.

9. Verwendung nach Anspruch 1, dadurch **gekennzeichnet**, daß das 15-Keto-PGE ein 15-Keto-16-mono- oder -dihalogen-PGE ist.

10. Verwendung nach Anspruch 9, dadurch **gekennzeichnet**, daß das 15-Keto-PGE ein 15-Keto-16-mono- oder -difluor-PGE ist.

11. Verwendung nach Anspruch 1, dadurch **gekennzeichnet**, daß das 15-Keto-PGE ein 15-Keto-16-mono- oder -dialkyl-PGE ist.

12. Verwendung nach Anspruch 11, dadurch **gekennzeichnet**, daß das 15-Keto-PGE ein 15-Keto-16-mono- oder -dimethyl-PGE ist.

13. Verwendung nach Anspruch 1, dadurch **gekennzeichnet**, daß das 15-Keto-PGE ein 6,15-Diketo-16-alkyl-PGE ist.

14. Verwendung nach Anspruch 1, dadurch **gekennzeichnet**, daß das 15-Keto-PGE ein 6,15-Diketomethyl-PGE ist.

15. Verwendung nach Anspruch 1, dadurch **gekennzeichnet**, daß das 15-Keto-PGE ein 13,14-Dihydro-6,15-diketo-16-alkyl-PGE ist.

16. Verwendung nach Anspruch 1, dadurch **gekennzeichnet**, daß das 15-Keto-PGE ein 13,14-Dihydro-6,15-diketo-16-methyl-PGE ist.

17. Verwendung nach Anspruch 1 für die Behandlung von oder die Prophylaxe gegen Krankheiten, die durch Erhöhung des Luftwegwiderstands verursacht werden.

18. Verwendung nach Anspruch 17, dadurch **gekennzeichnet**, daß die Krankheiten Bronchialasthma, infektiöse asthmatische Bronchitis oder chronische Bronchitis sind.

19. Verwendung nach Anspruch 17, dadurch **gekennzeichnet,** daß die Verabreichung der Zusammensetzung durch Sprühen, intravenöse Injektion, subkutane Injektion, überzogene Suppositorien oder durch Gurgeln erfolgt.

**Patentansprüche für folgende Vertragsstaaten : ES, GR**

1. Verfahren zur Herstellung einer Zusammensetzung, die für die Tracheobronchodilatation wirksam ist, dadurch **gekennzeichnet**, daß ein 15-Keto-PGE mit mindestens einem Substituenten an irgendeiner der 3-, 6-, 16-, 17-, 19- und 20-Stellungen und ein physiologisch annehmbaren Träger für ein Arzneimittel vermischt werden.

2. Verfahren nach Anspruch 1, dadurch **gekennzeichnet**, daß das 15-Keto-PGE durch die folgende Formel:

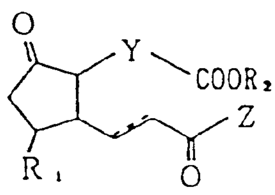

dargestellt wird, worin $R_1$ eine Hydroxylgruppe, eine Hydroxyalkylgruppe oder eine Alkylgruppe bedeutet; Y eine gegebenenfalls substituierte, gesättigte oder ungesättigte Kohlenwasserstoff-Gruppierung mit 2 bis 6 Kohlenstoffatomen, wovon mindestens eines eine Carbonylgruppe sein kann, bedeutet; Z eine gegebenenfalls substituierte, gesättigte oder ungesättigte Kohlenwasserstoff-Gruppierung, die eine gerade Kette mit einer Kohlenstoffzahl von 1 bis 10 oder ein Ring sein kann, bedeutet; und $R_2$ ein Wasserstoffatom, ein physiologisch annehmbares Salz oder einen Esterrest bedeutet.

3. Verfahren nach Anspruch 1, dadurch **gekennzeichnet,** daß die 15-Keto-PGEs 13,14-Dihydro-15-keto-PGEs sind.

4. Verfahren nach Anspruch 1, dadurch **gekennzeichnet**, daß die Carboxylgruppe in der Endstellung der α-Kette des 15-Keto-PGEs verestert ist.

5. Verfahren nach Anspruch 1, dadurch **gekennzeichnet**, daß die Bindung zwischen der 2-Stellung und der 3-Stellung des 15-Keto-PGEs eine Doppelbindung ist.

6. Verfahren nach Anspruch 1, dadurch **gekennzeichnet**, daß das 15-Keto-PGE eine Methylgruppe in der 3-Stellung besitzt.

7. Verfahren nach Anspruch 1, dadurch **gekennzeichnet,** daß das 15-Keto-PGE ein 6,15-Diketo-PGE ist.

8. Verfahren nach Anspruch 1, dadurch **gekennzeichnet**, daß das 15-Keto-PGE in der 20-Stellung mit einer $C_1$-$C_4$-Alkylgruppe oder Alkoxygruppe substituiert ist.

9. Verfahren nach Anspruch 1, dadurch **gekennzeichnet**, daß das 15-Keto-PGE ein 15-Keto-16-mono-oder -dihalogen-PGE ist.

10. Verfahren nach Anspruch 9, dadurch **gekennzeichnet**, daß das 15-Keto-PGE ein 15-Keto-16-mono-oder -difluor-PGE ist.

11. Verfahren nach Anspruch 1, dadurch **gekennzeichnet**, daß das 15-Keto-PGE ein 15-Keto-16-mono-oder -dialkyl-PGE ist.

12. Verfahren nach Anspruch 11, dadurch **gekennzeichnet,** daß das 15-Keto-PGE ein 15-Keto-16-mono-oder -dimethyl-PGE ist.

13. Verfahren nach Anspruch 1, dadurch **gekennzeichnet,** daß das 15-Keto-PGE ein 6,15-Diketo-16-alkyl - PGE ist.

14. Verfahren nach Anspruch 1, dadurch **gekennzeichnet**, daß das 15-Keto-PGE ein 6,15-Diketo-methyl-PGE ist.

15. Verfahren nach Anspruch 1, dadurch **gekennzeichnet,** daß das 15-Keto-PGE ein 13,14-Dihydro-6,15-diketo-16-alkyl-PGE ist.

16. Verfahren nach Anspruch 1, dadurch **gekennzeichnet**, daß das 15-Keto-PGE ein 13,14-Dihydro-6,15-diketo-16-methyl-PGE ist.

17. Verfahren nach Anspruch 1, dadurch **gekennzeichnet,** daß das Arzneimittel für die Behandlung von oder die Prophylaxe gegen Krankheiten, die durch Erhöhung des Luftwegwiderstands verursacht werden, bestimmt ist.

18. Verfahren nach Anspruch 17, dadurch **gekennzeichnet**, daß das Arzneimittel für die Behandlung oder

die Prophylaxe gegen Bronchialasthma, infektiöse asthmatische Bronchitis oder chronische Bronchitis bestimmt ist.

19. Verfahren nach Anspruch 17, dadurch **gekennzeichnet**, daß das Arzneimittel für die Verabreichung durch Sprühen, intravenöse Injektion, subkutane Injektion, überzogene Suppositorien oder durch Gurgeln geeignet ist.

## Revendications

### Revendications pour les Etats contractants suivants : AT, BE, CH, DE, FR, GB, IT, LI, LU, NL, SE

1. Utilisation d'une composition comprenant une 15-céto-PGE ayant au moins un substituant à l'une quelconque des positions 3, 6, 16, 17, 19 et 20 et un support physiologiquement acceptable pour la fabrication d'un médicament efficace pour la trachéobronchodilatation.

2. Utilisation selon la revendication 1, dans laquelle la 15-céto-PGE est représentée par la formule suivante :

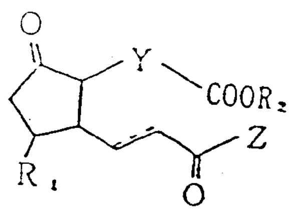

dans laquelle $R_1$ est un groupe hydroxyle, un groupe hydroxyalkyle ou un groupe alkyle; Y est une partie hydrocarbonée saturée ou insaturée, éventuellement substituée, ayant 2 à 6 atomes de carbone dont l'un au moins peut être un groupe carbonyle; Z est une partie hydrocarbonée saturée ou insaturée, éventuellement substituée, constituant une chaîne linéaire ayant 1 à 10 atomes de carbone ou un cycle; et $R_2$ est un atome d'hydrogène, un sel physiologiquement acceptable ou un radical d'ester.

3. Utilisation selon la revendication 1, dans laquelle les 15-céto-PGE sont des 13,14-dihydro-15-céto-PGE.

4. Utilisation selon la revendication 1, dans laquelle un groupe carboxyle en position terminale de la chaîne $\alpha$ de la 15-céto-PGE est estérifié.

5. Utilisation selon la revendication 1, dans laquelle la liaison entre la position 2 et la position 3 de la 15-céto-PGE est une double liaison.

6. Utilisation selon la revendication 1, dans laquelle la 15-céto-PGE possède un groupe méthyle à la position 3.

7. Utilisation selon la revendication 1, dans laquelle la 15-céto-PGE est une 6,15-dicéto-PGE.

8. Utilisation selon la revendication 1, dans laquelle la 15-céto-PGE est substituée à la position 20 avec un groupe alkyle ou un groupe alcoxy en $C_1$ à $C_4$.

9. Utilisation selon la revendication 1, dans laquelle la 15-céto-PGE est une 15-céto-16-mono ou dihalogéno-PGE.

10. Utilisation selon la revendication 9, dans laquelle la 15-céto-PGE est une 15-céto-16-mono ou difluoro-PGE.

11. Utilisation selon la revendication 1, dans laquelle la 15-céto-PGE est une 15-céto-16-mono ou dialkyl-PGE.

12. Utilisation selon la revendication 11, dans laquelle la 15-céto-PGE est une 15-céto ou 16-mono ou diméthyl-PGE.

13. Utilisation selon la revendication 1, dans laquelle la 15-céto-PGE est une 6,15-dicéto-16-alkyl-PGE.

14. Utilisation selon la revendication 1, dans laquelle la 15-céto-PGE est une 6,15-dicéto-méthyl-PGE.

15. Utilisation selon la revendication 1, dans laquelle la 15-céto-PGE est une 13,14-dihydro-6,15-dicéto-16-alkyl-PGE.

16. Utilisation selon la revendication 1, dans laquelle la 15-céto-PGE est une 13,14-dihydro-6,15-dicéto-16-méthyl-PGE.

17. Utilisation selon la revendication 1, pour le traitement des maladies causées par l'augmentation de la résistance des voies aériennes ou pour la protection contre celles-ci.

18. Utilisation selon la revendication 17, dans laquelle les maladies sont l'asthme bronchique, la bronchite asthmatique infectieuse ou la bronchite chronique.

19. Utilisation selon la revendication 17, dans laquelle l'administration de la composition s'effectue par pulvérisation, injection intraveineuse, injection sous-cutanée, suppositoire, enduction ou gargarisme.

**Revendications pour les Etats contractants suivant : ES, GR**

1. Procédé pour la fabrication d'une composition efficace pour la trachéobronchodilatation, comprenant le fait de mélanger une 15-céto-PGE ayant au moins un substituant à l'une quelconque des positions 3, 6, 16, 17, 19 et 20 et un support physiologiquement acceptable d'un médicament.

2. Procédé selon la revendication 1, dans lequel la 15-céto-PGE est représentée par la formule suivante :

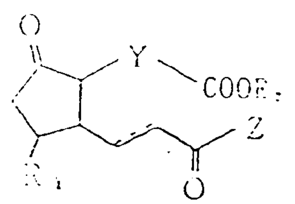

dans laquelle $R_1$ est un groupe hydroxyle, un groupe hydroxyalkyle ou un groupe alkyle; Y est une partie hydrocarbonée saturée ou insaturée, éventuellement substituée, ayant 2 à 6 atomes de carbone dont l'un au moins peut être un groupe carbonyle; Z est une partie hydrocarbonée saturée ou insaturée, éventuellement substituée, constituant une chaîne linéaire ayant 1 à 10 atomes de carbone ou un cycle; et $R_2$ est un atome d'hydrogène, un de ses sels ou un radical d'ester physiologiquement acceptables.

3. Procédé selon la revendication 1, dans lequel les 15-céto-PGE sont des 13,14-dihydro-15-céto-PGE.

4. Procédé selon la revendication 1, dans lequel un groupe carboxyle à la position terminale de la chaîne $\alpha$ de la 15-céto-PGE est estérifié.

5. Procédé selon la revendication 1, dans lequel la liaison entre les position 2 et 3 de la 15-céto-PGE est une double liaison.

6. Procédé selon la revendication 1, dans lequel la 15-céto-PGE possède un groupe méthyle à la position 3.

7. Procédé selon la revendication 1, dans lequel la 15-céto-PGE est une 6,15-dicéto-PGE.

8. Procédé selon la revendication 1, dans lequel la 15-céto-PGE est substituée à la position 20 par un groupe alkyle ou un groupe alcoxy en $C_1$ à $C_4$.

9. Procédé selon la revendication 1, dans lequel la 15-céto-PGE est une 15-céto-16-mono ou dihalogéno-PGE.

**10.** Procédé selon la revendication 9, dans lequel la 15-céto-PGE est une 15-céto-16-mono ou difluoro-PGE.

**11.** Procédé selon la revendication 1, dans lequel la 15-céto-PGE est une 15-céto-16-mono ou dialkyl-PGE.

**12.** Procédé selon la revendication 11, dans lequel la 15-céto-PGE est une 15-céto-16-mono ou diméthyl-PGE.

**13.** Procédé selon la revendication 1, dans lequel la 15-céto-PGE est une 6,15-dicéto-16-alkyl-PGE.

**14.** Procédé selon la revendication 1, dans lequel la 15-céto-PGE est une 6,15-dicéto-méthyl-PGE.

**15.** Procédé selon la revendication 1, dans lequel la 15-céto-PGE est une 13,14-dihydro-6,15-dicéto-16-alkyl-PGE.

**16.** Procédé selon la revendication 1, dans lequel la 15-céto-PGE est une 13,14-dihydro-6,15-dicéto-16-méthyl-PGE.

**17.** Procédé selon la revendication 1, dans lequel le médicament est destiné au traitement de maladies causées par l'augmentation de la résistance des voies aériennes ou à la protection contre celles-ci.

**18.** Procédé selon la revendication 17, dans lequel le médicament est destiné au traitement de l'asthme bronchique, de la bronchite asthmatique infectieuse ou de la bronchite chronique ou à la protection contre celles-ci.

**19.** Procédé selon la revendication 17, dans lequel le médicament est destiné à l'administration par pulvérisation, injection intraveineuse, injection sous-cutanée, Suppositoire, enduction ou gargarisme.

Synthetic Chart I

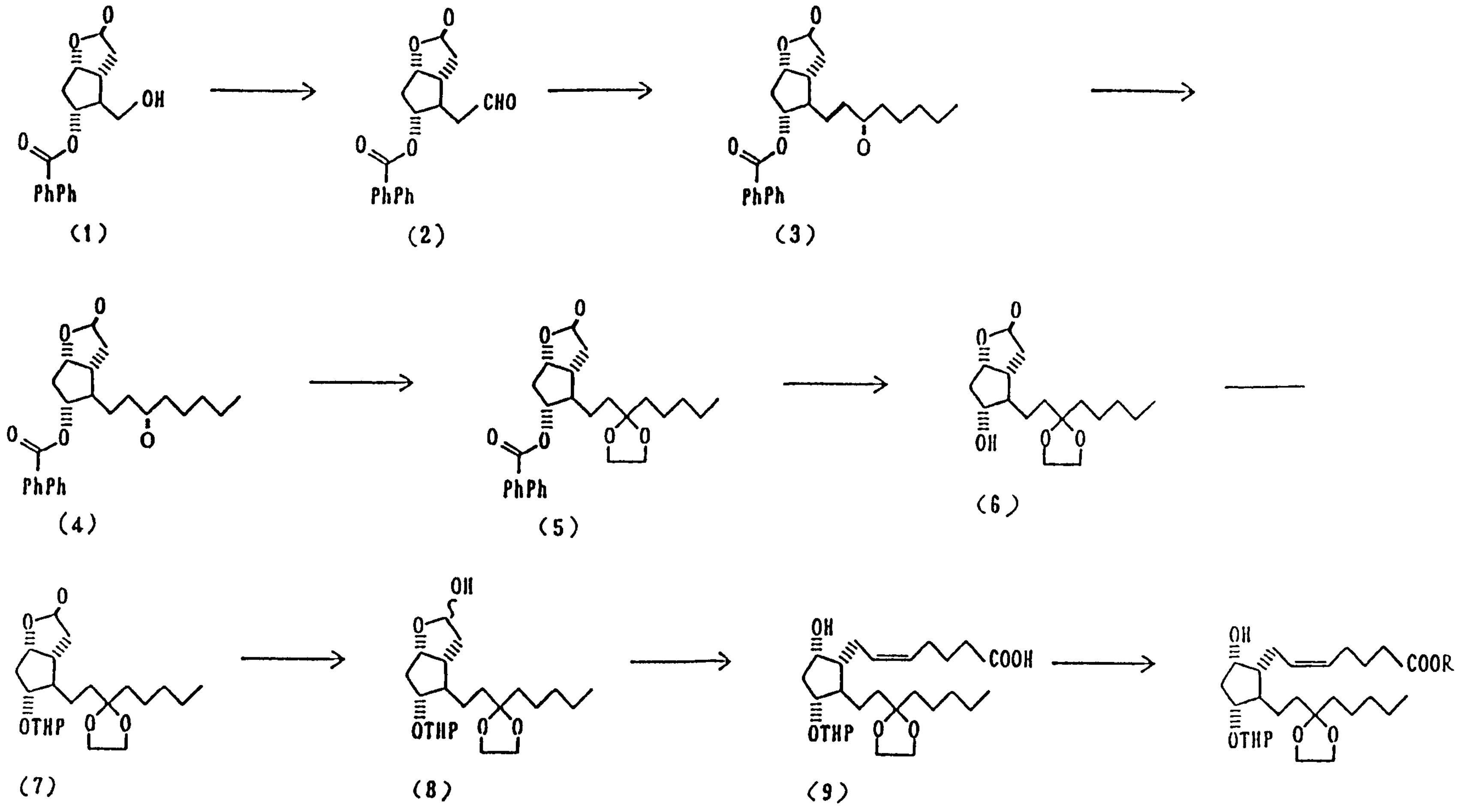

(1) (2) (3)

(4) (5) (6)

(7) (8) (9) (10)

PhPh= 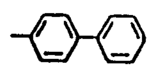 THP= 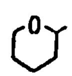

Synthetic Chart I (continued)

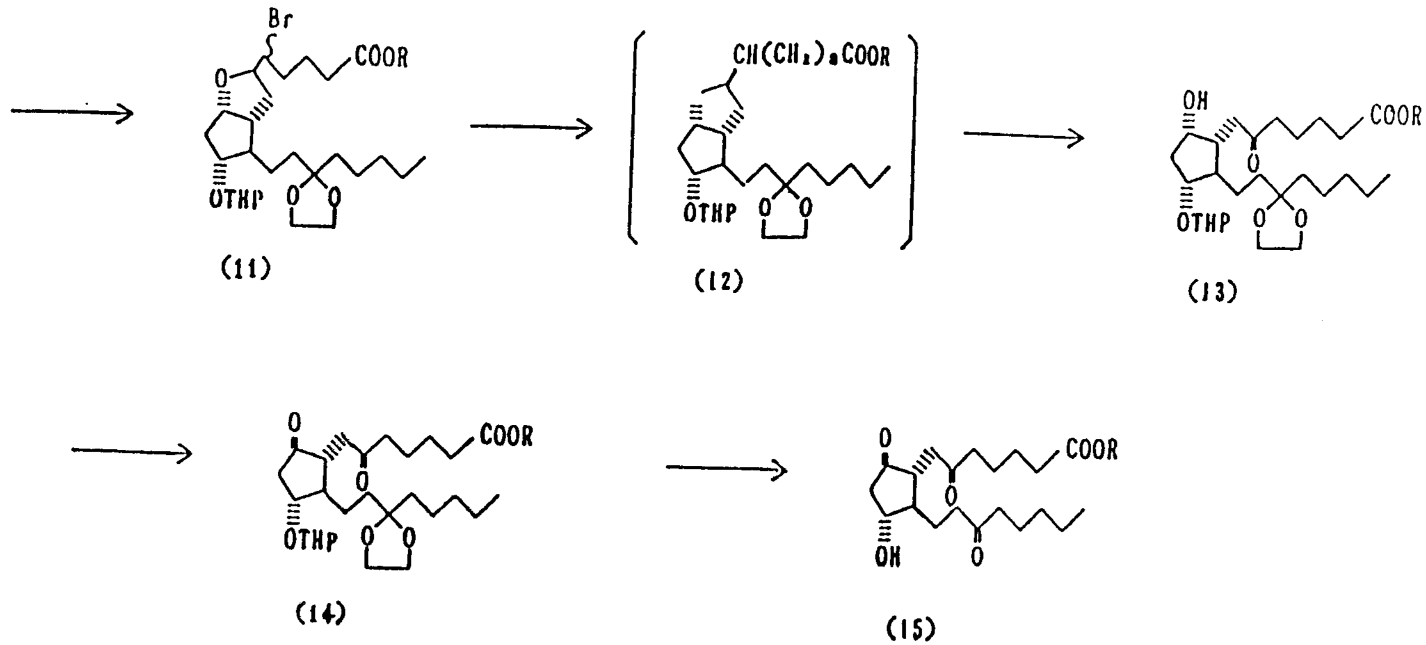

(11)

(12)

(13)

(14)

(15)

R:Et or Me

Synthetic Chart II

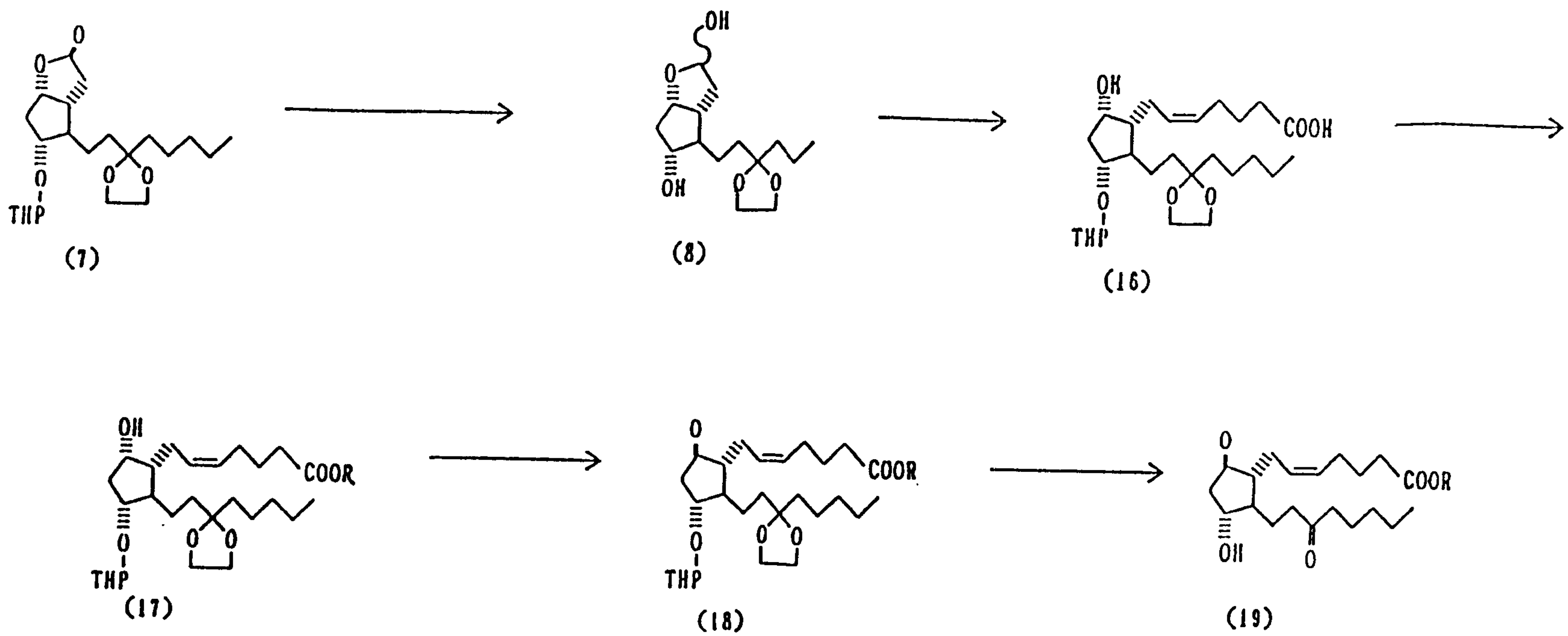

(7) (8) (16)

(17) (18) (19)

Syhthetic Chart III

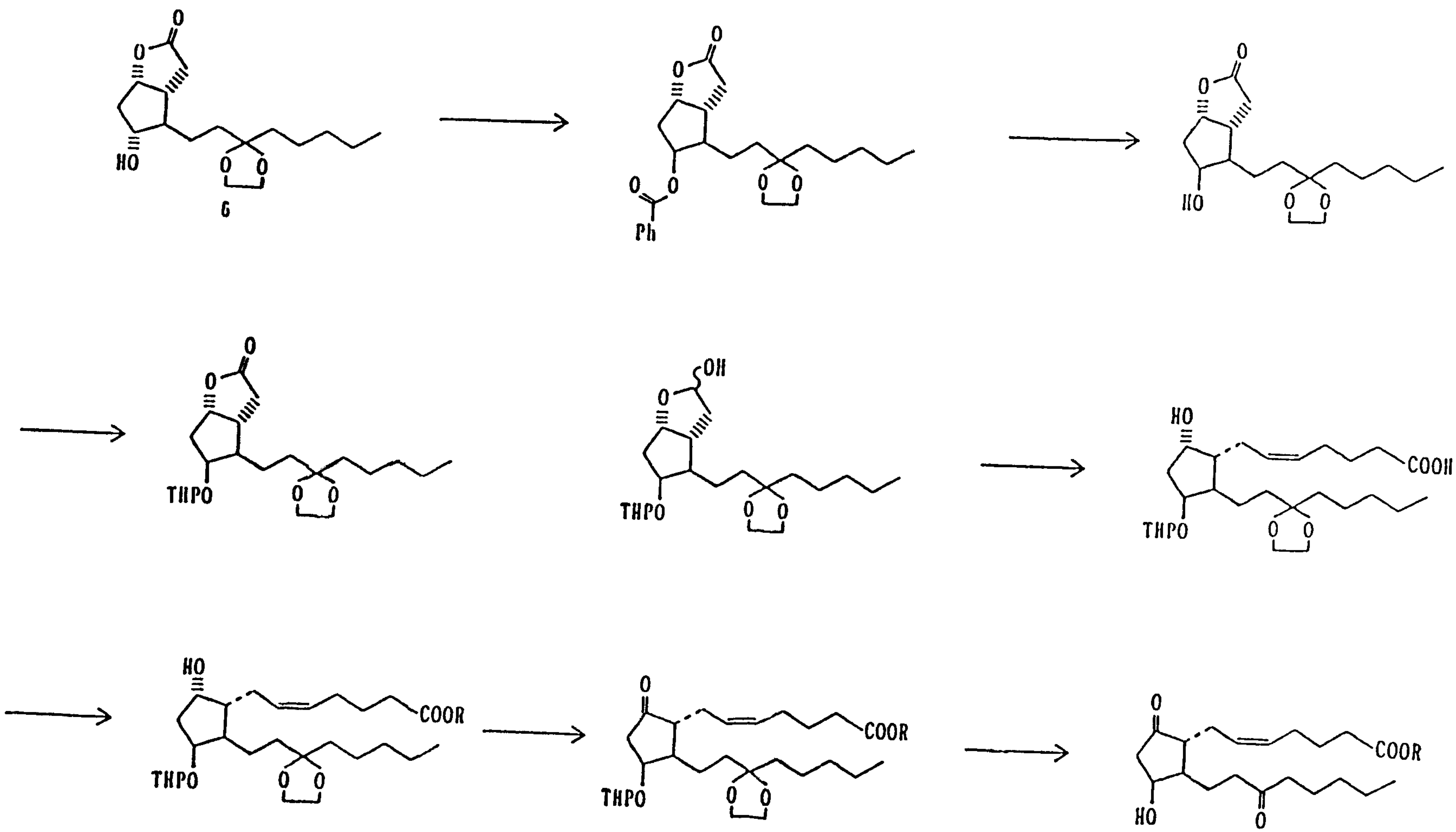

6